# EUROPEAN PATENT APPLICATION

(11) **EP 3 081 140 A1**
(43) Date of publication of application: **19.10.2016**
(21) Application number: 14869428.4
(22) Date of filing: 10.12.2014
(51) Int. Cl.: A61B 1/00

(54) **ENDOSCOPE TREATMENT INSTRUMENT**

(30) Priority: 11.12.2013 JP 2013256426
(71) Applicant: Olympus Corporation, Tokyo 192-8507 (JP)
(72) Inventor: ETO, Hirofumi, Tokyo 192-8507 (JP); UEMICHI, Katsuji, Tokyo 192-8507 (JP); MATSUNO, Kiyotaka, Tokyo 192-8507 (JP)
(74) Representative: Schicker, Silvia
(86) International application number: PCT/JP2014/082762
(87) International publication number: WO 2015/087939

(57) **Abstract**

This endoscope treatment instrument is provided with a sheath which is configured to be insertable into a channel of the endoscope, a treatment unit which is disposed at the leading end of the sheath, an elongate member which is inserted into the sheath so as to allow advancing and retracting, connects at the leading end to the treatment unit, and operates the treatment unit by advancing and retracting with respect to the sheath, and an operation unit which is provided on the base end of the sheath. The operation unit comprises an operation unit main body connected to the sheath, a slider provided movably along the axial direction of the sheath with respect to the operation unit main body and connected to the base end of the elongate member, and a constant load spring provided on the operation unit main body and biasing the slider towards the base end with a constant force regardless of the position of the slider.

## Description

### [Technical Field]

The present invention relates to an endoscope treatment tool that performs a treatment on tissue.

Priority is claimed on Japanese Patent Application No. 2013-256426, filed on December 11, 2013, the content of which is incorporated herein by reference.

### [Background Art]

Conventionally, while performing observation with an endoscope, various treatments are performed by inserting an endoscope treatment tool into a channel formed in the endoscope.

As this type of the endoscope treatment tool, for example, an ultrasonic puncture needle described in Patent Literature 1 is known.

The ultrasonic puncture needle is mainly consisted of a sheath, an operating portion arranged at a proximal end portion of the sheath, and a needle tube that is arranged to be inserted into the sheath in such a state as freely advancing/retreating in the sheath via the operating portion. The operating portion includes an operating portion body having a rod shape and attached to the proximal end portion of the sheath, and a slider movably disposed at an intermediate portion in a longitudinal direction of the operating portion body. The slider has a fixing screw for fixing to the operating portion body, and a proximal end portion of the needle tube is attached to the slider.

When the ultrasonic puncture needle configured as described above is used, the endoscope insertion portion of the ultrasonic endoscope is inserted from the mouth or the like into the body. A site to perform a biopsy is identified while observation is performed using an optical imaging system or an ultrasonic scanning system in the ultrasonic endoscope.

The slider is moved (pullback) towards the proximal end side with respect to the operating portion body, and the fixing screw is tightened in a state in which the needle tube is accommodated in the sheath. A user such as an operator grips the endoscope operating portion of the ultrasonic endoscope with one hand, and inserts the sheath of the ultrasonic puncture needle into the channel from a proximal end port provided on the endoscope operating portion. After projecting the sheath from the endoscope insertion portion, the user looses the fixing screws of the ultrasonic puncture needle with the other hand to protrude the needle tube from the distal end portion of the sheath by pushing the slider. The length of pushing the slider in order to protrude the needle tube is, for example, about 40 mm.

In this case, the user operates the slider during gripping the endoscope operating portion with one hand and gripping the operating portion of the ultrasonic puncture needle with the other hand. Therefore, the slider, which becomes an emphasis point of a force, and the coupling portion between the ultrasonic puncture needle and the proximal end port, which becomes a fulcrum point, are spaced at relatively distant interval and the operation is not stable.

After protruding the needle tube from the sheath, the distal end of the needle tube is made to reach the target tissue to perform a biopsy. Subsequently, the user connects a syringe or the like to the conduit of the needle tube to aspirate inside the needle tube, and cells of the target tissue are aspirated from the distal end of the needle tube.

To solve the problem that the force point and the fulcrum are spaced at relatively distant interval when operating the operating portion of the endoscopic treatment tool that is inserted into the channel of the endoscope, it is considered to use an operating portion that is used in a common micropipette or the like as the endoscope treatment tool. In this operating portion, the slider is provided so as to protrude towards more proximal end side than the proximal end face of the operating portion body, and a helical spring is provided for biasing the slider towards the proximal end side. The user, for example, grips the operating portion body with fingers of the index finger to the little finger of a hand, and pushes the slider towards the distal end side with the thumb against the biasing force of the helical spring to protrude the needle tube from the sheath.

When using this operating portion, the slider that becomes the main emphasis and the operating portion body that becomes the fulcrum point are relatively close to fit in one hand and it is possible to operate the operating portion of the endoscope treatment tool in stable condition. When the user releases the thumb from the slider, the needle tube is accommodated within the sheath by the biasing force of the helical spring.

### [Citation List]

### [Patent Literature]

[Patent Literature 1] Japanese Patent Publication No. 3890013

### [Summary of Invention]

### [Technical Problem to be solved by the Invention]

However, when using this operating unit, the reaction force received from the helical spring increases in accordance with the state that the slider is pushed, and it is difficult to hold the slider in a state in which the slider is most pressed.

In consideration of the above-described problems, the present invention is directed to provide an endoscope treatment tool that can be easily held in a state in which the slider is pushed.

### [Means for solving the Problem]

According to a first aspect of the present invention, an endoscope treatment instrument includes: a sheath configured to be capable of being inserted into a channel of an endoscope; a treatment portion provided at a distal end portion of the sheath; an elongate member inserted into the sheath so as to be capable of advancing and retracting, a distal end portion of the elongate member being connected to the treatment portion, the elongate member advancing and retracting with respect to the sheath to operate the treatment portion; and an operating portion provided on a proximal end portion of the sheath. The operating portion includes: an operating portion body connected to the sheath; a slider provided movably along a direction of an axial line of the sheath with respect to the operating portion body and connected to a proximal end portion of the elongate member; and a constant force spring portion provided on the operating portion body and biasing the slider towards a proximal end side with a constant force regardless of a position of the slider.

According to a second aspect of the present invention, in the endoscope treatment instrument according to the first aspect, the constant force spring portion may include: a spring member formed to have a long length; and an accommodating portion that accommodates a proximal end portion of the spring member, the accommodating portion may be disposed at a proximal end side of the operating portion body to protrude laterally from the operating portion body, and a distal end portion of the spring member may be extending towards a distal end side of the elongate member along the elongate member and is connected to one of the slider and the elongate member.

According to a third aspect of the present invention, in the endoscope treatment instrument according to the first aspect, the treatment portion and the elongate member may be configured as one structure of a needle tube, a through hole that communicates with a conduit line of the needle tube may be formed through the slider, a receiving surface may be disposed on the axis line of the sheath at a proximal end of the slider, and an opening of a proximal end portion of the through hole may be formed at a position deviated from the axis line of the sheath at the proximal end of the slider.

### [Advantageous Effects of Invention]

According to the endoscope treatment tool of the above aspects, it is possible to easily hold the endoscope treatment tool in a state in which the slider is pushed.

### [Brief Description of Drawings]

Fig. 1 is an explanatory view of an endoscope used in the first embodiment of the present invention.
Fig. 2 is a sectional view of a side of a puncture needle in accordance with the first embodiment of the present invention.
Fig. 3 is a cross-sectional view of a plane of the proximal end side of the puncture needle in accordance with the first embodiment of the present invention.
Fig. 4 is a rear view of the puncture needle in accordance with the first embodiment of the present invention.
Fig. 5 is a side view of the proximal end side of the puncture needle in accordance with the first embodiment of the present invention.
Fig. 6 is a perspective view of a main portion of the puncture needle in accordance with the first embodiment of the present invention.
Fig. 7 is a plan view of a locking member of the puncture needle in accordance with the first embodiment of the present invention.
Fig. 8 is a sectional view for explaining the operation of the puncture needle in accordance with the first embodiment of the present invention.
Fig. 9 is a sectional view of a side of the puncture needle in accordance with the first embodiment of the present invention.
Fig. 10 is a side view of the proximal end side of the puncture needle in accordance with the first embodiment of the present invention.
Fig. 11 is a plan view of the distal end side of a snare in accordance with a modification of the first embodiment of the present invention.

### [Description of Embodiments]

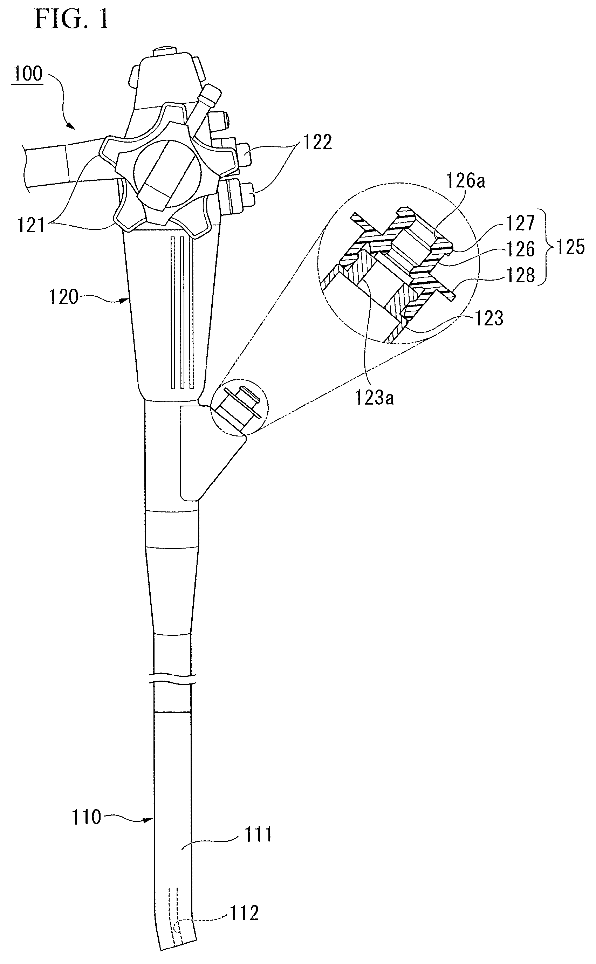

Hereinafter, an endoscope treatment tool in accordance with an embodiment of the present invention will be described with reference to Figs. 1 to 10, exemplifying the case in which the endoscopic treatment tool is a puncture needle (aspiration biopsy needle). Further, in all of the following drawings, for the purpose of easy understanding of the drawings, thicknesses of components or the ratio of dimensions may be appropriately varied.

First, an endoscope to be used with the puncture needle in accordance with the present embodiment will be described. In the following, although an example in which the endoscope is so-called a direct-view observable endoscope that can observe in the front side of the endoscope insertion portion will be described, the configuration of the endoscope is not limited to this. The endoscope may be so-called a side-view type endoscope that can observe a side of the endoscope insertion portion.

Fig. 1 is an explanatory view of an endoscope 100 used in the present embodiment. As illustrated in Fig.1, the endoscope 100 is an endoscope having a known configuration, and includes an endoscope insertion portion 110 that is elongate and flexible and an endoscope operating section 120. The endoscope operating portion 120 is provided on the proximal end portion of the endoscope insertion section 110.

A bending portion 111 that is capable of bending to be operated is provided at the distal end side of the endoscope insertion portion 110. An operation wire, which is not shown in the figures, is mounted on the bending portion 111. The operation wire is inserted through the endoscope insertion portion 110, and extends to the endoscope operating portion 120. A light guide and an imaging unit having a CCD are provided at the distal end surface of the endoscope insertion portion 110 in a state in which they are exposed, although not shown in the figures.

In the endoscope insertion portion 110, a channel 112 is formed along the endoscope insertion portion 110. The distal end portion of the channel 112 is opened to the distal end surface of the endoscope insertion portion 110.

An angle knob 121 and a switch 122 are provided in the endoscope operating portion 120. The angle knob 121 is provided for operating the above-described operation wire. By operating the angle knob 121, it is possible to bend the bending portion 111 in a desired direction. The switch 122 is provided for operating a light source, which is not shown in the figures, a monitor, the above-described imaging unit, or the like.

A forceps insertion port 123 is provided at the distal end side of the endoscope operating portion 120. An insertion hole 123a is formed through the forceps insertion port 123. The insertion hole 123a of the forceps insertion port 123 is communicated with the channel 112.

An adapter forceps plug 125 formed of a material having elasticity such as a rubber is fitted to the forceps insertion port 123. The adapter forceps plug 125 includes an adapter body 126, a locking portion 127, and a flange 128. The adapter body 126 is formed in a cylindrical shape. The locking portion 127 is provided at the proximal end portion of the outer peripheral surface of the adapter body 126. The flange 128 is provided at the intermediate portion in the longitudinal direction of the outer peripheral surface of the adapter body 126.

The inner diameter of the insertion hole 126a of the adapter body 126 is slightly smaller than the inner diameter of the insertion hole 123a of the forceps insertion port 123. A check valve, which is not shown in the figures, for preventing liquid from flowing to the outside from the side of the forceps insertion port 123 via the through hole 126a is provided on the adapter forceps plug 125.

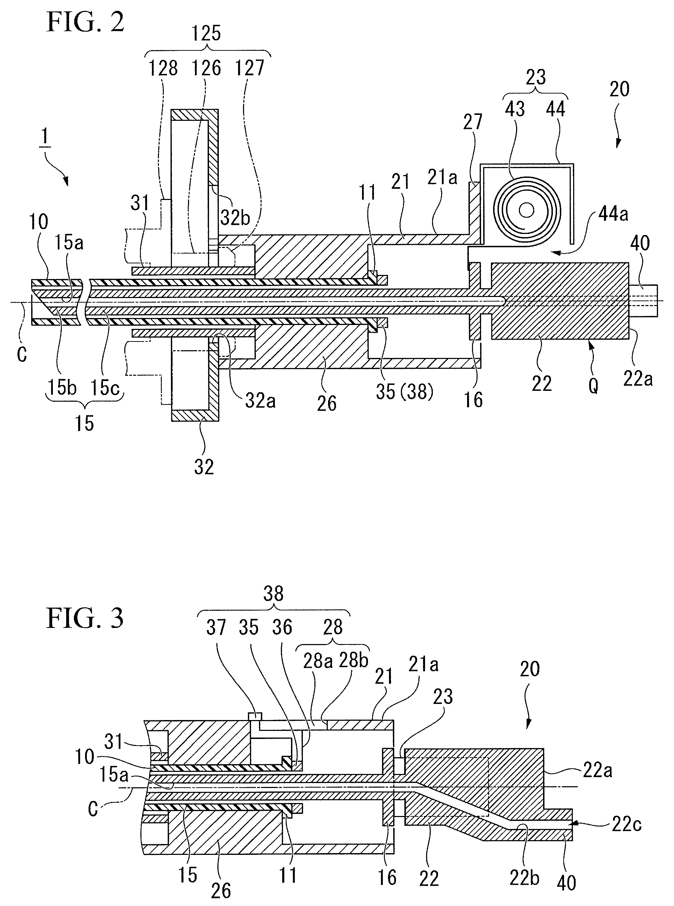

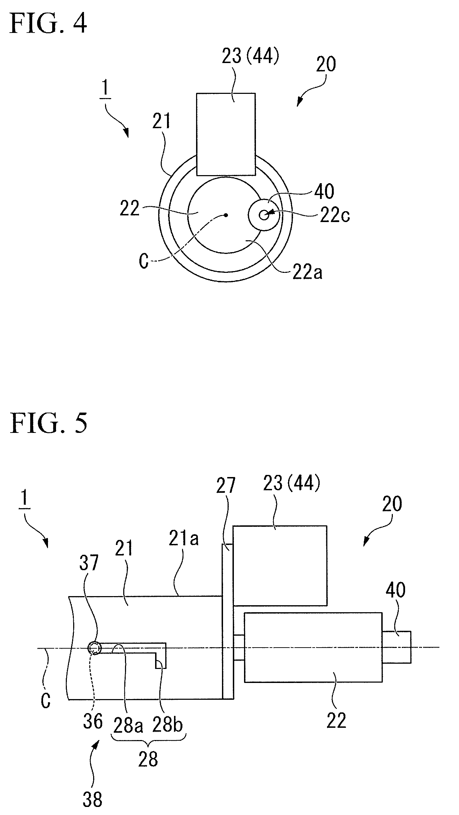

Fig. 2 is a sectional view of a side of a puncture needle 1 in accordance with the present embodiment. Fig. 3 is a cross-sectional view of a plane of the proximal end side of the puncture needle 1 in accordance with the present embodiment. Fig. 4 is a rear view of the puncture needle 1 in accordance with the present embodiment. As illustrated in Figs. 2 to 4, the puncture needle 1 in accordance with the present embodiment includes a sheath 10, a needle tube 15, and an operating portion 20. The needle tube 15 is movably inserted through the sheath 10. The operating portion 20 is provided at the proximal end portion of the sheath 10.

The sheath 10 has such an outer diameter as to be capable of inserting into the channel 112 of the endoscope 100. The sheath 10 may be formed of a polyetheretherketone (PEEK), a fluorine-based resin, an olefin-based resin, a urethane-based resin, a nylon-based (polyamide-based) resin, a metal coil, or the like. An enlarged diameter portion 11 whose outer diameter is enlarged is provided at the proximal end portion of the sheath 10.

The needle tube 15 is formed as a tube. A flange portion 16 is provided at the proximal end portion of the outer peripheral surface of the needle tube 15. The distal end of the needle tube 15 is formed sharply so as to pierce the tissue by being cut off obliquely to the axis (central axis) C of the sheath 10.

The material that forms the needle tube 15 and the flange portion 16 is a material having flexibility, and preferably having elasticity so as to easily restore the linear state even if being bent by an external force. As such a material, an alloy material such as stainless steel alloys, nickel titanium alloys, cobalt chromium alloys, or the like can be cited.

The needle tube 15 includes a treatment portion 15b and an elongated member 15c. The treatment portion 15b is a needle tip provided at the distal end portion of the sheath 10. The elongated member 15c is a needle body whose distal end portion is connected to the treatment portion 15b. The treatment portion 15b and the elongated member 15c are integrally formed in the needle tube 15 of the present embodiment. The elongated member 15c makes the treatment portion 15b operating by advancing and retracting relative to the sheath 10. That is, the elongated member 15c is a member for recovering the target tissue by the treatment portion 15b as described below.

The operating portion 20 includes an operating portion body 21, a slider 22, and a constant force spring portion 23. The operating portion body 21 is connected to the proximal end portion of the sheath 10. The slider 22 is connected to the proximal end portion of the needle tube 15. The constant force spring portion 23 is provided in the operating portion body 21.

The operating portion body 21 is formed in a cylindrical shape, and a reduced diameter portion 26 that protrudes radially inward is formed at the intermediate portion in the axial direction C of the inner circumferential surface of the operating portion body 21. At the proximal end portion of the outer peripheral surface (side surface) 21a of the operating portion body 21, a support portion 27 is provided that protrudes outward in the radial direction of the operating portion body 21. The operating portion body 21, the reduced diameter portion 26, and the support portion 27 are integrally formed of an ABS resin (a styrene-acrylonitrile-butadiene copolymer) or the like.

Fig. 5 is a side view of the proximal end side of the puncture needle 1 in accordance with the present embodiment. As shown in Figs. 3 and 5, a slit 28 is formed in the operating portion body 21. Slits 28 are formed so as to reach the inner peripheral surface from the outer peripheral surface 21 a. The slits 28 includes a slide slit 28a and a locking slit 28b. The slide slit 28a extends parallel to the axis C. The locking slit 28b communicates with the slide slit 28a, and extends from the proximal end portion of the slide slit 28a in the circumferential direction. Width of the slide slit 28a is equal to width of the locking slit 28b.

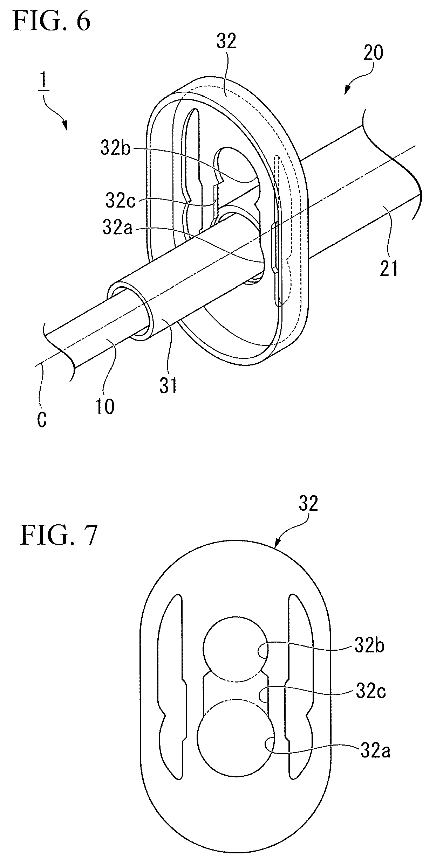

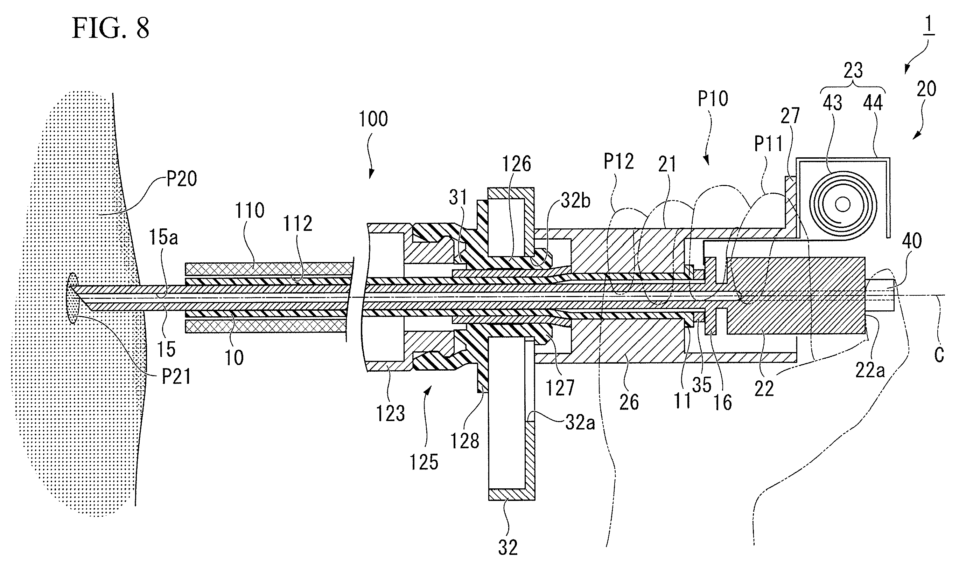

Fig. 6 is a perspective view of a main portion of the puncture needle 1 of the present embodiment. Fig. 7 is a plan view of a locking member 32 of the puncture needle 1 in accordance with the present embodiment. Fig. 8 is a sectional view for explaining the operation of the puncture needle 1 in accordance with the present embodiment. As shown in Figs. 2 to 6, a support pipe 31 extending towards the distal end side is fixed to the distal end surface of the reduced diameter portion 26 of the operating portion body 21. The support pipe 31 can be formed of a metal such as stainless steel. The sheath 10 is inserted into the support pipe 31. The outer diameter of the support pipe 31 is slightly smaller than the inner diameter of the insertion hole 123 a of the forceps insertion port 123 of the endoscope 100.

At the distal end portion of the operating portion body 21, the lock member 32 having a plate-like shape is provided slidably in a direction perpendicular to the axis C. As shown in Fig. 7, a large diameter hole 32a, a small diameter hole 32b, and a communication hole 32c are formed in the lock member 32. The small diameter hole 32b has an inner diameter that is smaller than the large diameter hole 32a. The communication hole 32c makes the large diameter hole 32a communicate with the small diameter hole 32b.

As shown in Figs. 2 and 6, the support pipe 31 can be inserted into the large diameter hole 32a, the small diameter hole 32b, and the communication hole 32c of the lock member 32. That is, in a state in which the support pipe 31 is inserted into a hole of the large diameter hole 32a, the small diameter hole 32b, and the communication hole 32c of the lock member 32, the lock member 32 can be slid in the direction perpendicular to the axis C relative to the operating portion body 21.

As shown in Fig. 2, when the lock member 32 is slid to the side of the small diameter hole 32b of the operating portion body 21, the large diameter hole 32a is positioned on the axis C. In this case, the lock member 32 is not engaged to the adapter forceps plug 125.

Meanwhile, the adapter body 126 of the adapter forceps plug 125 is inserted into the large diameter hole 32a of the lock member 32 that is slid to the side of the small diameter hole 32b, and as shown in Fig. 8, while the lock member 32 is contacting to the flange 128, the lock member 32 is slid to the side of the large diameter hole 32a with respect to the operating portion body 21. As a result, the edge portion of the small diameter hole 32b is engaged with the locking portion 127 and is restricted to move towards the proximal end side of the lock member 32 relative to the adapter forceps plug 125, and the lock member 32 is attached to the adapter forceps plug 125.

As shown in Figs. 2 and 3, the sheath 10 is inserted into the reduced diameter portion 26 of the operating portion body 21, and is fixed to the reduced diameter portion 26 with an adhesive or the like, which is not shown in the figures, in a state in which the enlarged diameter portion 11 is in contact with the proximal end surface of the reduced diameter portion 26.

A restricting member 35 having a ring like shape is disposed between the enlarged diameter portion 11 of the sheath 10 and the flange portion 16 of the needle tube 15. The needle tube 15 is inserted into the cylinder bore of the restricting member 35. As shown in Fig. 3, the restricting member 35 is connected to the first end of the coupling member 36. The second end of the coupling member 36 is inserted into the slit 28 and is drawn to the outside. A knob 37 whose diameter is larger than the width of the slit 28 is fixed to the second end of the coupling member 36.

A state switching portion 38 is configured by the slit 28, the restricting member 35, the coupling member 36, and the knob 37.

The slider 22 is formed to have a cylindrical shape, and the outer diameter of the slider 22 is smaller than the inner diameter of the operating portion body 21 on more proximal end side than the reduced diameter portion 26. A receiving surface 22a located on the axis line C is provided at the proximal end of the slider 22. The proximal end side of the receiving surface 22a is exposed to the outside. A through hole 22b is formed thorough the slider 22. The distal end portion of the through hole 22b is located on the axis C and communicates with the conduit 15a of the needle tube 15. The through hole 22b is bent so as to apart from the axis C with going towards the proximal end side, and is open to the proximal end of the slider 22. That is, the opening 22c of the proximal end portion of the through hole 22b is provided at a position displaced from the axis C.

In the present embodiment, a coupling portion 40, which extends to radially outward and to the proximal end side, is provided at a part of the circumferential direction of the slider 22, and the proximal end side of the through-holes 22b is formed inside the coupling portion 40. Thereby, the opening 22c of the through hole 22b is disposed at more proximal end side than the receiving surface 22a.

A known syringe or the like is detachably constructed at the proximal end portion of the coupling portion 40.

As shown in Fig. 2, the constant force spring portion 23 includes a spring member 43 and a drum (housing portion) 44. The spring member 43 is formed in an elongated shape such as a linear shape or a strip shape. The drum 44 accommodates the proximal end portion of the spring member 43.

As the spring material 43, a known constant force spring (such as CONSTON that is the trademark of the constant force spring developed by SUNCO SPRING CO., LTD.), or the like can be preferably used. The drum 44 is formed, for example, such that the outer shape thereof has a rectangular parallelepiped box shape and an open 44a is formed on one surface thereof. As shown in Fig. 4, the drum 44 protrudes laterally from the outer peripheral surface 21 a of the operating portion body 21 to the proximal end side of the operating portion body 21 (radially outward of the operating portion body 21), and, as shown in Fig. 2, an opening 44a is disposed so as to face the side of the axis C and is fixed to the support portion 27 by an adhesive or screws.

The proximal end portion of the spring member 43 is wound in the drum 44. The distal end portion of the spring member 43 extends towards the distal end side along the needle tube 15 through the opening 44a of the drum 44, and is connected to the needle tube 15 through the flange portion 16. The distal end portion of the spring member 43 is connected to the needle tube 15 by welding or the like, although not shown in the figures. The spring member 43 biases the slider 22 towards the proximal end side through the needle tube 15 with a constant force (including a substantially constant force) regardless of the position of the slider 22 relative to the operating portion body 21 in the direction of the axial C.

As shown in Fig. 3, a state in which the restricting member 35 contacts to the proximal end portion of the sheath 10 and the second end of the coupling member 36 is disposed at the distal end side of the slide slit 28a of the slits 28 is referred to as a non-restricted state of the state switching portion 38. In the non-restricted state of the state switching portion 38, the slider 22 can be pushed relative to the operating portion body 21 (can be moved towards the distal end side) from a state in which the needle tube 15 is accommodated in the sheath 10 as shown in Fig. 2 until the flange portion 16 of the needle tube 15 comes into contact with restricting member 35 as shown in Fig. 8.

Thus, when the state switching portion 38 is made to be in a non-restricted state, the slider 22 can move to more distal end side than the position Q that the slider 22 is most pulled back as shown in Fig. 2 (a predetermined position) relative to the operating portion body 21. That is, the slider 22 is movable along the direction of the axis C relative to the operating portion body 21. In both cases when the slider 22 is pushed and when the slider 22 is retracted, the proximal end surface of the slider 22 is more protruding towards the proximal end side than the proximal end surface of the operating portion body 21 is protruding. When the slider 22 is pushed, the distal end side of the slider 22 is disposed inside the operating portion body 21.

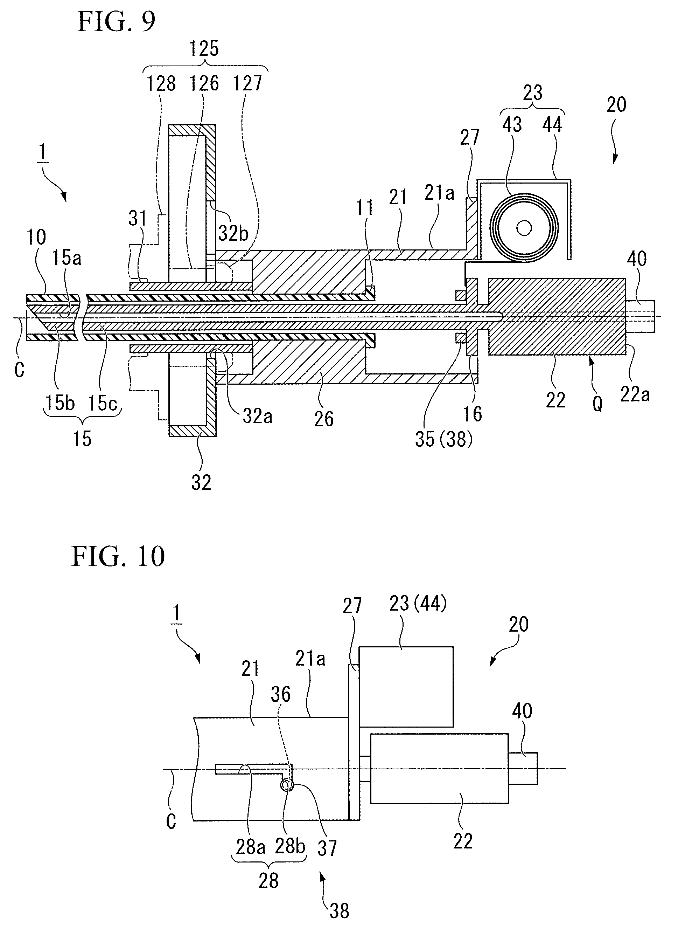

Fig. 9 is a sectional view of a side of the puncture needle 1 in accordance with the present embodiment. Fig. 10 is a side view of the proximal end side of the puncture needle 1 in accordance with the present embodiment. As shown in Fig. 10, a state in which the second end of the coupling member 36 is disposed at the locking slit 28b of the slits 28 is referred to as a restricted state of the state switching portion 38. In the restricted state of the state switching portion 38, the restricting member 35 is moved towards more proximal end side than in the non-restricted state as shown in Fig. 9, and the coupling member 36 is locked in the direction of the axis C by the locking slit 28b as shown in Fig. 10. Since the distal end surface of the flange portion 16 is in contact with the restricting member 35 at the position Q where the slider 22 is the most retracted to the proximal end side, the slider 22 is restricted to move towards more distal end side than the position Q relative to the operating portion body 21.

In this way, by operating the knob 37 to change the position where the second end of the coupling member 36 is located within the slits 28, it is possible to switch the state of the state switching portion 38 between the restricted state and the non-restricted state.

Next, the operation of the puncture needle 1 constructed as described above will be described. In the following descriptions, the treatment of a biopsy to collect a lesion, which is located deep in the lung, as a target tissue will be explained for example.

When the user manipulates the switch 122 of the endoscope 100 to operate the light source, the illumination light emitted from the light source illuminates the front of the endoscope insertion portion 110, guided by the light guide. Images in the front of the endoscope insertion portion 110 obtained by the imaging unit is displayed on the monitor. While checking the image displayed on the monitor, the user inserts the endoscope insertion portion 110 of the endoscope 100 into the body of the patient. While operating the angle knob 121 to bend the bending portion 111 as appropriate, the user inserts the endoscope insertion portion 110. The site to perform a biopsy is made to face the distal end surface of the endoscope insertion portion 110.

Next, the needle tube 15 is accommodated in the sheath 10 by pulling back the slider 22 of the puncture needle 1, and the locking member 32 is slid to the side of the small diameter hole 32b. The state switching section 38 is left to be in the restricted state. It is preferable that a stylet is inserted into the pipe line 15a of the needle tube 15 from the side of the through hole 22b of the slider 22.

The user grips the endoscope operating section 120 with one hand, and inserts the sheath 10 of the puncture needle 1 and the support pipe 31 into the insertion hole 126a of the adapter forceps plug 125 and the insertion hole 123a of the forceps insertion port 123 of the endoscope 100. At this time, the check valve of the adapter forceps plug 125 is moved by the sheath 10. A gap between the adapter forceps plug 125 and the support pipe 31 is sealed in watertight by the elastic force of the adapter forceps plug 125.

Since the outer diameter of the support pipe 31 and the inner diameter of the insertion hole 123a of the forceps insertion port 123 is set as described above, the support pipe 31 slides inside the insertion hole 123a and the support pipe 31 is reliably supported to the forceps insertion cap 123.

The adapter body 126 of the adapter forceps plug 125 is inserted into the large diameter hole 32a of the lock member 32, and the lock member 32 is attached to the adapter forceps plug 125 by sliding the lock member 32 to the side of the large diameter hole 32a. The state of the state switching unit 38 is set to a non-restricted state.

As shown in Fig. 8, the user grips a portion of the operating portion body 21 at more distal end side than the support portion 27 by the index finger P11 to the little finger P12 of the other hand P10, and puts the thumb P13 on the receiving surface 22a of the slider 22. The slider 22 is pushed with the other hand P10 and the needle tube 15 is protruded from the sheath 10.

Since the receiving surface 22a and the needle tube 15 of the slider 22 are disposed on the axis C, the force to the distal end side that is affected on the receiving surface 22a can be effectively transferred to the needle tube 15. Since the coupling portion 40 and the opening 22c of the proximal end portion of the through hole 22b are provided at a position deviated from the axis C, the user can operate the receiving surface 22a with no hindrance.

Since the slider 22 that becomes the power point and the operating portion body 21 that becomes the fulcrum point are fit inside the other hand P10 of the user, it is possible to perform stably the operation of the operating portion 20. By multiplying the index finger P11 to the constant force spring portion 23 through the support portion 27 when pressing the receiving surface 22a of the slider 22 with the thumb P13, the user can reliably press the receiving surface 22a towards the distal end side while supporting the operating portion body 21 towards the proximal end side with the index finger P11.

Since the force that the slider 22 is biased towards the proximal end side by the constant force spring 23 is fixed, the reaction force that thumb P 13 receives from the receiving surface 22a is not increased even if the slider 22 is pushed.

The distal end of the needle tube 15 is pierced into the tissue P20 and pushed into the target tissue P21 to perform the biopsy. The organization that enters the pipe line 15a of the needle tube 15 and is not a biopsy target is extruded by the stylet, and the stylet is pulled from the needle tube 15 and the slider 22. A syringe or the like is attached to the coupling portion 40 of the slider 22 to suck inside the through hole 22b and the pipe 15a by operating the syringe.

The target tissue P21 enters into the syringe through inside of the conduit line 15a and inside of the through hole 22b. When the required amount of the target tissue P21 is collected, the slider 22 is pulled back to accommodate the needle tube 15 into the sheath 10. Thereby, the needle tube 15 exits from the tissue P20. By sliding the lock member 32 to the side of the small diameter hole 32b, the engagement between the adapter forceps plug 125 and the locking member 32 is released. The sheath 10 of the puncture needle 1 is pulled out from the channel 112 of the endoscope 100.

The endoscope 100 is pulled from the patient, the required action is performed, and the series of procedures end.

As described above, according to the puncture needle 1 of the present embodiment, the slider 22 is biased towards the proximal end side with a constant force by the constant force spring 23, regardless of the position of the slider 22 in the direction of the axial direction C. Therefore, even if the length to push the slider 22 is long, the force required to push the slider 22 does not increase and the slider 22 can be easily held in a state in which the slider 22 is pushed. When the thumb P13 is released from the slider 22, the slider 22 is moved to the position Q in the proximal end side by the constant force spring 23. Since the user needs only to perform the operation of pushing the slider 22 and the operation of pulling back the slider 22 is performed automatically, it is possible to improve the operability of the operating portion 20. The user releases the thumb P13 from the slider 22, thereby, it is possible to accommodate the needle tube 15 into the sheath 10 by the constant force spring 23. Thereby, it is possible to prevent the channel 112 of the endoscope 100 from being damaged by the needle tube 15.

The drum 44 is provided at the proximal end side of the operating portion body 21 so as to protrude towards the side direction from the outer peripheral surface 21 a of the operating portion body 21. Accordingly, the user grips a part of more distal end side than the support portion 27 in the operating portion body 21 by the index finger P11 to little finger P12, and the index finger P11 can be applied to the constant force spring 23 through the support portion 27. Accordingly, when pressing the receiving surface 22a of the slider 22 with the thumb P13, the user securely grips the operating portion body 21 by the index finger P11 to the little finger P12, and the operating portion body 21 can be supported towards the proximal end side by the index finger P11.

Since the receiving surface 22a of the slider 22 is disposed on the axial line C, the force to the distal end side affected on the receiving surface 22a can be effectively transferred to the needle tube 15. Since the coupling portion 40 and the opening 22c of the proximal end portion of the through hole 22b is provided at a position deviated from the axis C, it is possible to prevent a state in which the coupling portion 40 and the opening 22c become a trouble when operating the receiving surface 22a. By making the state of the state switching portion 38 in a non-restricted state, the slider 22 can be moved towards more distal end side than the position Q and it is possible to freely perform the operation in which the needle tube 15 is accommodated into the sheath 10 or the needle tube 15 is projected from the sheath 10. Meanwhile, by making the state of the state switching portion 38 in the restricted state, it is possible to prevent the slider 22 from moving towards more distal end side than the position Q and the needle tube 15 from protruding from the sheath 10 unintentionally.

While an embodiment of the present invention has been described above in detail with reference to the drawings, the concrete configuration is not limited to this embodiment. Modifications, combinations, omissions, or the like of the configurations are included without departing from the substance of the present invention.

For example, in the present embodiment, an example in which the distal end portion of the spring member 43 is connected to the needle tube 15 has been described, but the distal end portion of the spring member 43 may be connected to the slider 22.

The state switching unit 38 may not be provided in the operating portion 20.

In the present embodiment, an example in which the endoscopic treatment instrument is the puncture needle 1 has been described. However, the endoscope treatment tool is not limited to this, and may be an endoscope ligating device or the like for introducing a snare, forceps, a clip unit into the body.

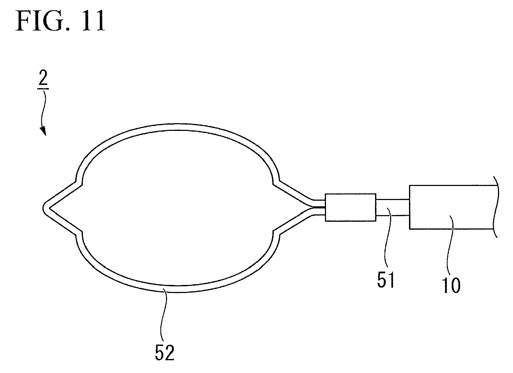

For example, when the endoscopic treatment instrument is the snare 2 shown in Fig. 11, the operating wire 51 inserted into the sheath 10 corresponds to the elongated member and the loop wire 52 connected to the distal end portion of the operating wire 51 corresponds to the treatment portion.

Hereinabove, while the embodiment of the present invention has been described, the technical scope of the present invention is not limited to the above-mentioned embodiment but combinations of the components of the embodiment may be varied, various modifications may be added to the components, or the components may be deleted without departing from the spirit of the present invention. The present invention is not limited to the above description but is limited only by the scope of the accompanying claims.

### [Industrial Applicability]

It is possible to provide an endoscope treatment instrument capable of being easily held in a state in which a slider is pushed into the endoscope treatment instrument.

### [Reference Signs List]

- 1: puncture needle (endoscope treatment instrument)
- 2: snare (endoscope treatment instrument)
- 10: sheath
- 15: needle tube
- 15a: conduit line
- 15b: treatment portion
- 15c: elongate member
- 20: operating portion
- 21: operating portion body
- 21a: outer peripheral surface (side surface)
- 22: slider
- 22a: receiving surface
- 22b: through hole
- 22c: opening
- 23: constant load spring portion
- 43: spring member
- 44: dram (accommodating portion)
- 51: operating wire (elongate member)
- 52: loop wire (treatment portion)
- 100: endoscope
- 112: channel
- C: axis line

## Claims

1. An endoscope treatment instrument comprising:
a sheath configured to be capable of being inserted into a channel of an endoscope;
a treatment portion provided at a distal end portion of the sheath;
an elongate member inserted into the sheath so as to be capable of advancing and retracting, a distal end portion of the elongate member being connected to the treatment portion, the elongate member advancing and retracting with respect to the sheath to operate the treatment portion; and
an operating portion provided on a proximal end portion of the sheath,
wherein the operating portion includes:
an operating portion body connected to the sheath;
a slider provided movably along a direction of an axial line of the sheath with respect to the operating portion body and connected to a proximal end portion of the elongate member; and
a constant force spring portion provided on the operating portion body and biasing the slider towards a proximal end side with a constant force regardless of a position of the slider.

2. The endoscope treatment instrument according to claim 1, wherein
the constant force spring portion includes:
a spring member formed to have a long length; and
an accommodating portion that accommodates a proximal end portion of the spring member,
the accommodating portion is disposed at a proximal end side of the operating portion body to protrude laterally from the operating portion body, and
a distal end portion of the spring member is extending towards a distal end side of the elongate member along the elongate member and is connected to one of the slider and the elongate member.

3. The endoscope treatment instrument according to claim 1, wherein
the treatment portion and the elongate member are configured as one structure of a needle tube,
a through hole that communicates with a conduit line of the needle tube is formed through the slider,
a receiving surface is disposed on the axis line of the sheath at a proximal end of the slider, and
an opening of a proximal end portion of the through hole is formed at a position deviated from the axis line of the sheath at the proximal end of the slider.
